# EUROPEAN PATENT SPECIFICATION

(11) **EP 1 651 270 B1**
(45) Date of publication and mention of the grant of the patent: **21.03.2007**
(21) Application number: 04741130.1
(22) Date of filing: 17.07.2004
(51) Int. Cl.: A61K 45/06, A61K 31/46, A61P 11/00

(54) **MEDICAMENTS FOR INHALATION COMPRISING BETAMIMETICS AND AN ANTICHOLINERGIC**
MEDIKAMENTE ZUR INHALATION MIT BETAMIMETIKA UND EINEM ANTICHOLINERGIKUM
MEDICAMENTS DESTINES A L'INHALATION CONTENANT DES BETAMIMETIQUES ET UN ANTICHOLINERGIQUE

(30) Priority: 29.07.2003 EP 03017163
(43) Date of publication of application: 03.05.2006
(60) Divisional application: 06122278.2
(73) Proprietor: Boehringer Ingelheim International GmbH, 55216 Ingelheim am Rhein (DE); Boehringer Ingelheim Pharma GmbH & Co.KG, 55216 Ingelheim am Rhein (DE)
(72) Inventor: MEADE, Christopher, John, Montague, 88437 MASELHEIM (DE); PAIRET, Michel, 88400 BIBERACH (DE); PIEPER, Michael, P., 88400 BIBERACH (DE)
(74) Representative: Hammann, Heinz
(86) International application number: PCT/EP2004/008030
(87) International publication number: WO 2005/014044

(56) References cited:
- WO-A-01/04118
- WO-A-03/024452
- DE-A- 10 216 333

## Description

The present invention relates to novel pharmaceutical compositions based on beta₂ agonists and salts of an anticholinergic, processes for preparing them and their use in the treatment of respiratory complaints.

### Description of the invention

The present invention relates to novel pharmaceutical compositions based on beta₂ agonists and salts of an anticholinergic **1,** processes for preparing them and their use in the treatment of respiratory complaints.

Within the scope of the present invention the anticholinergic agents used are the salts of formula **1** 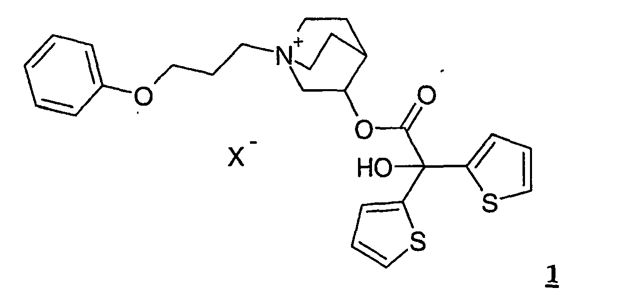 wherein
- X⁻: denotes an anion with a single negative charge, preferably an anion selected from the group consisting of fluoride, chloride, bromide, iodide, sulphate, phosphate, methanesulphonate, nitrate, maleate, acetate, citrate, fumarate, tartrate, oxalate, succinate, benzoate and p-toluenesulphonate, optionally in the form of the racemates, the enantiomers, and the hydrates thereof.

Preferably, the salts of formula **1** are used wherein
- X⁻: denotes an anion with a single negative charge selected from among the fluoride, chloride, bromide, 4-toluenesulphonate and methanesulphonate, preferably bromide, optionally in the form of the racemates, the enantiomers, and the hydrates thereof.

Most preferably, the salts of formula **1** are used wherein
- X⁻: denotes an anion with a single negative charge selected from among the chloride, bromide and methanesulphonate, preferably bromide, optionally in the form of the racemates, the enantiomers, and the hydrates thereof.

Particularly preferred according to the invention is the salt of formula **1** wherein X⁻ denotes bromide.

Of particular interest according to the invention are the enantiomers of formula **1-en** 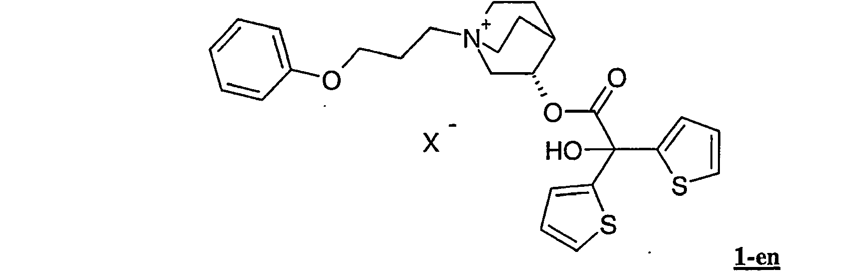 wherein X - may have the meanings an mentioned hereinbefore.

Surprisingly, an unexpectedly beneficial therapeutic effect can be observed in the treatment of inflammatory and/or obstructive diseases of the respiratory tract if the anticholinergic of formula **1** is used with one or more betamimetics **2.**

This effect may be observed both when the two active substances are administered simultaneously in a single active substance formulation and when they are administered successively in separate formulations. According to the invention, it is preferable to administer the two active substance ingredients simultaneously in a single formulation.

In the pharmaceutical combinations mentioned above the active substances may be combined in a single preparation or contained in two separate formulations. Pharmaceutical compositions which contain the active substances **1** and **2** in a single preparation are preferred according to the invention.

According to the instant invention preferred beta₂ agonists **2** in the combinations according to the invention are selected from the group consisting of albuterol, bambuterol, bitolterol, broxaterol, carbuterol, clenbuterol, fenoterol, formoterol, hexoprenaline, ibuterol, isoetharine, isoprenaline, levosalbutamol, mabuterol, meluadrine, metaproterenol, orciprenaline, pirbuterol, procaterol, reproterol, rimiterol, ritodrine, salmeterol, salmefamol, soterenot, sulphonterol, tiaramide, terbutaline, tolubuterol, CHF-1035, HOKU-81, KUL-1248, 3-(4-{6-[2-Hydroxy-2-(4-hydroxy-3-hydroxymethyl-phenyl)-ethylamino]-hexyloxy}-butyl)-benzenesulfoneamide, 5-[2-(5,6-Diethyl-indan-2-ylamino)-1-hydroxy-ethyl]-8-hydroxy-1*H*-quinolin-2-one, 4-hydroxy-7-[2-{ [2-{ [3-(2-phenylethoxy)propylJsulphonyl}ethyl]-amino}ethyl]-2(3H)-benzothiazolone, 1-(2-fluoro-4-hydroxyphenyl)-2-[4-(1-benzimidazolyl)-2-methyl-2-butylamino]ethanol, 1-[3-(4-methoxybenzyl-amino)-4-hydroxyphenyl]-2-[4-(1-benzimidazolyl)-2-methyl-2-butylamino]ethanol, 1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-hydroxy-3-oxo-4H-1,4-benzoxazin-8-yl]-2-[3-(4-methoxyphenyl)-2-methyl-2-propylamino]ethanol, 1-[2H-5-hydroxy-3-oxo-4H-1,4-benzoxazin-8-yl]-2-[3-(4-n-butyloxyphenyl)-2-methyl-2-propylamino]ethanol, 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol, 5-hydroxy-8-(1-hydroxy-2-isopropylaminobutyl)-2H-1,4-benzoxazin-3-(4H)-one, 1-(4-amino-3-chloro-5-trifluormethylphenyl)-2-tert.-butylamino)ethanol and 1-(4-ethoxycarbonylamino-3-cyano-5-fluorophenyl)-2-(tert.-butylamino)ethanol, optionally in the form of the racemates, the enantiomers, the diastereomers and optionally the pharmacologically acceptable acid addition salts and the hydrates thereof.

According to the instant invention more preferred beta₂ agonists **2** are selected from the group consisting of bambuterol, bitolterol, carbuterol, clenbuterol, fenoterol, formoterol, hexoprenaline, ibuterol, pirbuterol, procaterol, reproterol, salmeterol, sulphonterol, terbutaline, tolubuterol, 3-(4-{ 6-[2-Hydroxy-2-(4-hydroxy-3-hydroxymethyl-phenyl)-ethylamino]-hexyloxy}-butyl)-benzenesulfoneamide, 5-[2-(5,6-Diethyl-indan-2-ylamino)-1-hydroxy-ethyl]-8-hydroxy-1*H*-quinolin-2-one , 4-hydroxy-7-[2-{ [2-{ [3-(2-phenylethoxy)propyl]sulphonyl}ethyl]-amino}ethyl]-2(3H)-benzothiazolone, 1-(2-fluoro-4-hydroxyphenyl)-2-[4-(1-benzimidazolyl)-2-methyl-2-butylamino]ethanol, 1-[3-(4-methox ybenzyl-amino)-4-hydroxyphenyl]-2-[4-(1-benzimidazolyl)-2-methyl-2-butylamino]ethanol, 1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-hydroxy-3-oxo-4H-1,4-benzoxazin-8-yl]-2-[3-(4-methoxyphenyl)-2-methyl-2-propylamino]ethanol, 1-[2H-5-hydroxy-3-oxo-4H-1,4-benzoxazin-8-yl]-2-[3-(4-n-butyloxyphenyl)-2-methyl-2-propylamino]ethanol, 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol, 5-hydroxy-8-(1-hydroxy-2-isopropylaminobutyl)-2H-1,4-benzoxazin-3-(4H)-one, 1-(4-amino-3-chloro-5-trifluormethylphenyl)-2-tert.-butylamino)ethanol and 1-(4-ethoxycarbonylamino-3-cyano-5-fluorophenyl)-2-(tert.-butylamino)ethanol, optionally in the form of the racemates, the enantiomers, the diastereomers and optionally the pharmacologically acceptable acid addition salts and the hydrates thereof.

More preferably, the betamimetics **2** used as within the compositions according to the invention are selected from among fenoterol, formoterol, salmeterol, 3-(4-{6-[2-Hydroxy-2-(4-hydroxy-3-hydroxymethyl-phenyl)-ethylamino]-hexyloxy }-butyl)-benzenesulfoneamide, 5-[2-(5,6-Diethyl-indan-2-ylamino)-1-hydroxy-ethyl]-8-hydroxy-1*H*-quinolin-2-one , 1-[3-(4-methoxybenzyl-amino)-4-hydroxyphenyl]-2-[4-(1-benzimidazolyl)-2-methyl-2-butylamino]ethanol, 1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-hydroxy-3-oxo-4H-1,4-benzoxazin-8-yl]-2-[3-(4-methoxyphenyl)-2-methyl-2-propylamino]ethanol, 1-[2H-5-hydroxy-3-oxo-4H-1,4-benzoxazin-8-yl]-2-[3-(4-n-butyloxyphenyl)-2-methyl-2-propylamino]ethanol, 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol, optionally in the form of the racemates, the enantiomers, the diastereomers and optionally the pharmacologically acceptable acid addition salts thereof, and the hydrates thereof. Of the betamimetics mentioned above the compounds formoterol, salmeterol, 3-(4-{6-[2-Hydroxy-2-(4-hydroxy-3-hydroxymethyl-phenyl)-ethylamino]-hexyloxy}-butyl)-benzenesulfoneamide, and 5-[2-(5,6-Diethyl-indan-2-ylamino)-1-hydroxy-ethyl]-8-hydroxy-1*H*-quinolin-2-one are particularly preferred, optionally in the form of the racemates, the enantiomers, the diastereomers and optionally the pharmacologically acceptable acid addition salts thereof, and the hydrates thereof. Of the betamimetics mentioned above the compounds formoterol and salmeterol are particularly preferred, optionally in the form of the racemates, the enantiomers, the diastereomers and optionally the pharmacologically acceptable acid addition salts thereof, and the hydrates thereof.

Examples of pharmacologically acceptable acid addition salts of the betamimetics **2** according to the invention are the pharmaceutically acceptable salts which are selected from among the salts of hydrochloric acid, hydrobromic acid, sulphuric acid, phosphoric acid, methanesulphonic acid, acetic acid, fumaric acid, succinic acid, lactic acid, citric acid, tartaric acid, 1-hydroxy-2-naphthalenecarboxylic acid, 4-phenylcinnamic acid, 5-(2.4-difluorophenyl)salicylic acid or maleic acid. If desired, mixtures of the abovementioned acids may also be used to prepare the salts **2.**

According to the invention, the salts of the betamimetics **2** selected from among the hydrochloride, hydrobromide, sulphate, phosphate, fumarate, methanesulphonate, 4-phenylcinnamate, 5-(2.4-difluorophenyl)salicylate, maleate and xinafoate are preferred. Particularly preferred are the salts of **2** in the case of salmeterol selected from among the hydrochloride, sulphate, 4-phenylcinnamate, 5-(2.4-difluorophenyl)salicylate and xinafoate, of which the 4-phenylcinnamate, 5-(2.4-difluorophenyl)salicylate and especially xinafoate are particularly important. Particularly preferred are the salts of **2** in the case of formoterol selected from the hydrochloride, sulphate and fumarate, of which the hydrochloride and fumarate are particularly preferred. Of exceptional importance according to the invention is formoterol fumarate.

Salts of salmeterol, formoterol, 3-(4-{6-[2-Hydroxy-2-(4-hydroxy-3-hydroxymethylphenyl)-ethylamino]-hexyloxy}-butyl)-benzenesulfoneamide, and 5-[2-(5,6-Diethyl-indan-2-ylamino)-1-hydroxy-ethyl]-8-hydroxy-1*H*-quinolin-2-one , are preferably used as the betamimetics **2** according to the invention. Of particular importance according to the invention are salmeterol and formoterol salts. Any reference to the term betamimetics **2** also includes a reference to the relevant enantiomers or mixtures thereof.

In the pharmaceutical compositions according to the invention, the compounds **2** may be present in the form of their racemates, enantiomers or mixtures thereof. The separation of the enantiomers from the racemates may be carried out using methods known in the art (e.g. by chromatography on chiral phases, etc.) If the compounds **2** are used in the form of their enantiomers, it is particularly preferable to use the enantiomers in the R configuration at the C-OH group.

As an example, any reference to the most preferred compounds **2** according to the invention, the salts of salmeterol and formoterol, also includes the relevant enantiomeric salts of *R*-salmeterol, S-salmeterol, *R,R-*formoterol, *S,S*-formoterol, *R,S*-formoterol, *S,R-*formoterol and the mixtures thereof, while the enantiomeric salts of *R*-salmeterol and *R,R-*formoterol are of particular importance. The compounds **2** may also be present according to the invention in the form of the hydrates or solvates thereof.

Where the present invention refers to betamimetics which are not in the form of salts, this is indicated by a reference to compounds **2'**. For example, the preferred betamimetics **2'** according to the invention which are not in salt form include the free base of formoterol, salmeterol whereas the particularly preferred compounds **2** according to the invention are salmeterol xinafoate or formoterol fumarate.

Within the scope of the present invention the betamimetics **2** may possibly also be referred to as sympathomimetics or beta-2-agonists (β₂-agonists). All these terms are to be regarded as interchangeable for the purposes of the present invention.

In one aspect the present invention relates to the abovementioned pharmaceutical compositions which contain, in addition to therapeutically effective quantities of **1** and **2**, a pharmaceutically acceptable carrier. In another aspect the present invention relates to the abovementioned pharmaceutical compositions which do not contain any pharmaceutically acceptable carrier in addition to therapeutically effective quantities of **1** and **2.**
The present invention also relates to the use of therapeutically effective quantities of the salts **1** for preparing a pharmaceutical composition containing betamimetics **2** for treating inflammatory or obstructive diseases of the respiratory tract. Preferably, the present invention relates to the abovementioned use for preparing a pharmaceutical composition for treating asthma or COPD.

Within the scope of the present invention the compounds **1** and **2** may be administered simultaneously or successively, while it is preferable according to the invention to administer compounds **1** und **2** simultaneously.

The present invention further relates to the use of therapeutically effect amounts of salts **1** and betamimetics **2** for treating inflammatory or obstructive respiratory complaints, particularly asthma or COPD.

The proportions in which the active substances **1** and **2** may be used in the active substance combinations according to the invention are variable. Active substances **1** and **2** may possibly be present in the form of their solvates or hydrates. Depending on the choice of the compounds **1** and **2**, the weight ratios which may be used within the scope of the present invention vary on the basis of the different molecular weights of the various salt forms. The pharmaceutical combinations according to the invention may contain **1** and **2'** generally in ratios by weight ranging from 1:5 to 500:1, preferably from 1:10 to 400:1.

The weight ratios specified below were based on the bromide of compound **1** and the free bases **2**' of the betamimetics salmeterol and formoterol which are particularly preferred according to the invention.

The pharmaceutical combinations according to the invention may contain **1** and **2'** in the case of formoterol, for example, in ratios by weight ranging from 1:10 to 300:1, preferably from 1:5 to 200:1, preferably 1:3 to 150:1, more preferably from 1:2 to 100:1.
For example, without restricting the scope of the invention thereto, preferred combinations of **1** and **2** according to the invention may contain the bromide **1** and formoterol 2**'** in the following weight ratios: 1:5, 1:4, 1:3, 1:2, 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 21:1, 22:1, 23:1, 24:1, 25:1, 26:1, 27:1, 28:1, 29:1, 30:1, 31:1, 32:1, 33:1, 34:1, 35:1, 36:1, 37:1, 38:1, 39:1,40:1,41:1,42:1, 43:1, 44:1, 45:1, 46:1, 47:1, 48:1, 49:1, 50:1, 51:1, 52:1, 53:1, 54:1, 55:1, 56:1, 57:1, 58:1, 59:1, 60:1, 61:1, 62:1, 63:1, 64:1, 65:1, 66:1, 67:1, 68:1, 69:1, 70:1, 71:1, 72:1, 73:1, 74:1, 75:1, 76:1, 77:1, 78:1, 79:1, 80:1, 81:1, 82:1, 83:1, 84:1, 85:1, 86:1, 87:1, 88:1, 89:1, 90:1, 91:1, 92:1, 93:1, 94:1, 95:1, 96:1, 97:1, 98:1, 99:1, 100:1.

The pharmaceutical compositions according to the invention containing the combinations of **1** and **2** are normally administered so that the bromide **1** and formoterol **2'** are present together in doses of 5 to 5000µg, preferably from 10 to 2000µg, more preferably from 15 to 1000µg, better still from 20 to 800µg per single dose.

For example, combinations of **1** and **2** according to the invention contain a quantity of the bromide of formula **1** and formoterol **2'** such that the total dosage per single dose is about 10µg, 15µg, 20µg, 25µg, 30µg, 35µg, 45µg, 50µg, 55µg, 60µg, 65µg, 70µg, 75µg, 80µg, 85µg, 90µg, 95µg, 100µg, 105µg, 110µg, 115µg, 120µg, 125µg, 130µg, 135µg, 140µg, 145µg, 150µg, 155µg, 160µg, 165µg, 170µg, 175µg, 180µg, 185µg, 190µg, 195µg, 200µg, 205µg, 210µg, 215µg, 220µg, 225µg, 230µg, 235µg, 240µg, 245µg, 250µg, 255µg, 260µg, 265µg, 270µg, 275µg, 280µg, 285µg, 290µg, 295µg, 300µg, 305µg, 310µg, 315µg, 320µg, 325µg, 330µg, 335µg, 340µg, 345µg, 350µg, 355µg, 360µg, 365µg, 370µg, 375µg, 380µg, 385µg, 390µg, 395µg, 400µg, 405µg, 410µg, 415µg, 420µg, 425µg, 430µg, 435µg, 440µg, 445µg, 450µg, 455µg, 460µg, 465µg, 470µg, 475µg, 480µg, 485µg, 490µg, 495µg, 500µg, 505µg, 510µg, 515µg, 520µg, 525µg, 530µg, 535µg, 540µg, 545µg, 550µg, 555µg, 560µg, 565µg, 570µg, 575µg, 580µg, 585µg, 590µg, 595µg, 600µg, 605µg, 610µg, 615µg, 620µg, 625µg, 630µg, 635µg, 640µg, 645µg, 650µg, 655µg, 660µg, 665µg, 670µg, 675µg, 680µg, 685µg, 690µg, 695µg, 700µg, 705µg, 710µg, 715µg, 720µg, 725µg, 730µg, 735µg, 740µg, 745µg, 750µg, 755µg, 760µg, 765µg, 770µg, 775µg, 780µg, 785µg, 790µg, 795µg, 800µg, 805µg, 810µg, 815µg, 820µg, 825µg, 830µg, 835µg, 840µg, 845µg, 850µg, 855µg, 860µg, 865µg, 870µg, 875µg, 880µg, 885µg, 890µg, 895µg, 900µg, 905µg, 910µg, 915µg, 920µg, 925µg, 930µg, 935µg, 940µg, 945µg, 950µg, 955µg, 960µg, 965µg, 970µg, 975µg, 980µg, 985µg, 990µg, 995µg, 1000µg, 1005µg, 1010µg, 1015µg, 1020µg, 1025µg, 1030µg, 1035µg, 1040µg, 1045µg, 1050µg, 1055µg, 1060µg, 1065µg, 1070µg, 1075µg, 1080µg, 1085µg, 1090µg, 1095µg, 1100µg or similar. It is clear to anyone skilled in the art that the suggested dosages per single dose specified above are not to be regarded as being limited to the numerical values actually stated. Fluctuations of about ± 2.5 µg, particularly in the decimal range, are also included, as will be apparent to the skilled man. In these dosage ranges, the active substances **1** and **2'** may be present in the weight ratios given above.

For example, without restricting the scope of the invention thereto, the combinations in which the bromide is used as the salt **1** and in which **2** denotes formoterol fumarate is used as a preferred combination of **1** and **2**, the pharmaceutical compositions according to the invention may contain for instance the following quantities for each single dose: 20µg of **1** and 2.9µg of **2**, 20µg of **1** and 5.7µg of **2**, 20µg of **1** and 11.5µg of **2**, 20µg of **1** and 17.2µg of **2**, 20µg of **1** and 22.9µg of **2**, 20µg of **1** and 28.5µg of **2**, 40µg of **1** and 2.9µg of **2**, 40µg of **1** and 5.7µg of **2**, 40µg of **1** and 11.5µg of **2**, 40µg of **1** and 17.2µg of **2**, 40µg of **1** and 22.9µg of **2**, 40µg of **1** and 28.5µg of **2**, 60µg of **1** and 2.9µg of **2**, 60µg of **1** and 5.7µg of **2**, 60µg of **1** and 11.5µg of **2**, 60µg of **1** and 17.2µg of **2**, 60µg of **1** and 22.9µg of **2**, 60µg of **1** and 28.5µg of **2**, 100µg of **1** and 2.9µg of **2**, 100µg of **1** and 5.7µg of **2**, 100µg of **1** and 11.5µg of **2**, 100µg of **1** and 17.2µg of **2**, 100µg of **1** and 22.9µg of **2**, 100µg of **1** and 28.5µg of **2**, 200µg of **1** and 2.9µg of **2**, 200µg of **1** and 5.7µg of **2**, 200µg of **1** and 11.5µg of **2**, 200µg of **1** and 17.2µg of **2**, 200µg of **1** and 22.9µg of **2**, 200µg of **1** and 28.5µg of **2**, 300µg of **1** and 2.9µg of **2**, 300µg of **1** and 5.7µg of **2**, 300µg of **1** and 11.5µg of **2**, 300µg of **1** and 17.2µg of **2**, 300µg of **1** and 22.9µg of **2**, 300µg of **1** and 28.5µg of **2**, 400µg of **1** and 2.9µg of **2**, 400µg of **1** and 5.7µg of 2, 400µg of **1** and 11.5µg of **2**, 400µg of **1** and 17.2µg of **2**, 400µg of **1** and 22.9µg of **2**, 400µg of **1** and 28.5µg of **2**, 500µg of **1** and 2.9µg of **2**, 500µg of **1** and 5.7µg of **2**, 500µg of **1** and 11.5µg of **2**, 500µg of **1** and 17.2µg of **2**, 500µg of **1** and 22.9µg of **2**, 500µg of **1** and 28.5µg of **2**, 600µg of **1** and 2.9µg of **2**, 600µg of **1** and 5.7µg of **2**, 600µg of **1** and 11.5µg of **2**, 600µg of **1** and 17.2µg of **2**, 600µg of **1** and 22.9µg of **2**, 600µg of **1** and 28.5µg of **2**, 700µg of **1** and 2.9µg of **2**, 700µg of **1** and 5.7µg of **2**, 700µg of **1** and 11.5µg of **2**, 700µg of **1** and 17.2µg of **2**, 700µg of **1** and 22.9µg of **2**, 700µg of **1** and 28.5µg, of **2**, 800µg of **1** and 2.9µg of **2**, 800µg of **1** and 5.7µg of **2**, 800µg of **1** and 11.5µg of **2**, 800µg of **1** and 17.2µg of **2**, 800µg of **1** and 22.9µg of **2**, 800µg of **1** and 28.5µg of **2**, 900µg of **1** and 2.9µg of **2**, 900µg of **1** and 5.7µg of **2,** 900µg of **1** and 11.5µg of **2,** 900µg of **1** and 17.2µg of **2,** 900µg of **1** and 22.9µg of **2,** 900µg of **1** and 28.5µg of **2**, 1000µg of **1** and 2.9µg of **2,** 1000µg of **1** and 5.7µg of **2,** 1000µg of **1** and 11.5µg of **2,** 1000µg of **1** and 17.2µg of **2,** 1000µg of **1** and 22.9µg of **2,** 1000µg of **1** and 28.5µg of **2.**

For example, the active substance combinations according to the invention may contain the bromide **1** and **2'** , in the case of salmeterol, in ratios by weight in the range from about 1:30 to 400:1, preferably 1:25 to 200:1, preferably 1:20 to 100:1, more preferably from 1:15 to 50:1.

For example, without restricting the scope of the invention thereto, preferred combinations of **1** and **2** according to the invention may contain the bromide of formula **1** and salmeterol **2'** in the following ratios by weight: 1:15, 1:14, 1:13,1:12, 1:11, 1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 21:1, 22:1, 23:1, 24:1, 25:1, 26:1, 27:1, 28:1, 29:1, 30:1, 31:1, 32:1, 33:1, 34:1, 35:1.

The pharmaceutical compositions according to the invention containing the combinations of **1** and **2** are usually administered so that the bromide of formula **1** and salmeterol **2'** are present together in dosages of 5 to 5000µg, preferably from 10 to 2000µg, more preferably from 15 to 1000µg, even more preferably from 20 to 800µg per single dose.

For example, combinations of **1** and **2** according to the invention contain an amount of the bromide of formula **1** and salmeterol **2'** such that the total dosage per single dose is about 15µg, 20µg, 25µg, 30µg, 35µg, 45µg, 50µg, 55µg, 60µg, 65µg, 70µg, 75µg, 80µg, 85µg, 90µg, 95µg, 100µg, 105µg, 110µg, 115µg, 120µg, 125µg, 130µg, 135µg, 140µg, 145µg, 150µg, 155µg, 160µg, 165µg, 170µg, 175µg, 180µg, 185µg, 190µg, 195µg, 200µg, 205µg, 210µg, 215µg, 220µg, 225µg, 230µg, 235µg, 240µg, 245µg, 250µg, 255µg, 260µg, 265µg, 270µg, 275µg, 280µg, 285µg, 290µg, 295µg, 300µg, 305µg, 310µg, 315µg, 320µg, 325µg, 330µg, 335µg, 340µg, 345µg, 350µg, 355µg, 360µg, 365µg, 370µg, 375µg, 380µg, 385µg, 390µg, 395µg, 400µg, 405µg, 410µg, 415µg, 420µg, 425µg, 430µg, 435µg, 440µg, 445µg, 450µg, 455µg, 460µg, 465µg, 470µg, 475µg, 480µg, 485µg, 490µg, 495µg, 500µg, 505µg, 510µg, 515µg, 520µg, 525µg, 530µg, 535µg, 540µg, 545µg, 550µg, 555µg, 560µg, 565µg, 570µg, 575µg, 580µg, 585µg, 590µg, 595µg, 600µg, 605µg, 610µg, 615µg, 620µg, 625µg, 630µg, 635µg, 640µg, 645µg, 650µg, 655µg, 660µg, 665µg, 670µg, 675µg, 680µg, 685µg, 690µg, 695µg, 700µg, 605µg, 610µg, 615µg, 620µg, 625µg, 630µg, 635µg, 640µg, 645µg, 650µg, 655µg, 660µg, 665µg, 670µg, 675µg, 680µg, 685µg, 690µg, 695µg, 700µg, 705µg, 710µg, 715µg, 720µg, 725µg, 730µg, 735µg, 740µg, 745µg, 750µg, 755µg, 760µg, 765µg, 770µg, 775µg, 780µg, 785µg, 790µg, 795µg, 800µg, 805µg, 810µg, 815µg, 820µg, 825µg, 830µg, 835µg, 840µg, 845µg, 850µg, 855µg, 860µg, 865µg, 870µg, 875µg, 880µg, 885µg, 890µg, 895µg, 900µg, 905µg, 910µg, 915µg, 920µg, 925µg, 930µg, 935µg, 940µg, 945µg, 950µg, 955µg, 960µg, 965µg, 970µg, 975µg, 980µg, 985µg, 990µg, 995µg, 1000µg, 1005µg, 1010µg, 1015µg, 1020µg, 1025µg, 1030µg, 1035µg, 1040µg, 1045µg, 1050µg, 1055µg, 1060µg, 1065µg, 1070µg, 1075µg, 1080µg, 1085µg, 1090µg, 1095µg, 1100µg, 1105µg, 1110µg, 1115µg, 1120µg, 1125µg, 1130µg, 1135µg, 1140µg, 1145µg, 1150µg, 1155µg, 1160µg, 1165µg, 1170µg, 1175µg, 1180µg, 1185µg, 1190µg, 1195µg, 1200µg, 1205µg, 1210µg, 1215µg, 1220µg, 1225µg, 1230µg, 1235µg, 1240µg, 1245µg, 1250µg, 1255µg, 1260µg, 1265µg, 1270µg, 1275µg, 1280µg, 1285µg, 1290µg, 1295µg, 1300µg, 1305µg, 1310µg, 1315µg, 1320µg, 1325µg, 1330µg, 1335µg, 1340µg, 1345µg, 1350µg, 1355µg, 1360µg, 1365µg, 1370µg, 1375µg, 1380µg, 1385µg, 1390µg, 1395µg, 1400µg or similar. It is clear to anyone skilled in the art that the suggested dosages per single dose specified above are not to be regarded as being limited to the numerical values actually stated. Fluctuations of about ± 2.5 µg, particularly in the decimal range, are also included, as will be apparent to the skilled man. In these dosage ranges, the active substances **1** and **2'** may be present in the weight ratios given above.

For example, without restricting the scope of the invention thereto, the combinations in which the bromide is used as the salt **1** and in which **2** denotes salmeterol xinafoate is used as a preferred combination of **1** and **2**, the pharmaceutical compositions according to the invention may contain for instance the following quantities for each single dose: 20µg of **1** and 18.2µg of **2**, 20µg of **1** and 36.3µg of **2**, 20µg of **1** and 72.6µg of **2**, 20µg of **1** and 108.9µg of **2**, 20µg of **1** and 145.2µg of **2**, 20µg of **1** and 290.4µg of **2**, 40µg of **1** and 18.2µg of **2**, 40µg of **1** and 36.3µg of **2**, 40µg of **1** and 72.6µg of **2**, 40µg of **1** and 108.9µg of **2**, 40µg of **1** and 145.2µg of **2**, 40µg of **1** and 290.4µg of **2**, 60µg of **1** and 18.2µg of **2,** 60µg of **1** and 36.3µg of **2**, 60µg of **1** and 72.6µg of **2**, 60µg of **1** and 108.9µg of **2**, 60µg of **1** and 145.2µg of **2**, 60µg of **1** and 290.4µg of **2**, 100µg of **1** and 18.2µg of **2**, 100µg of **1** and 36.3µg of **2,** 100µg of **1** and 72.6µg of **2**, 100µg of **1** and 108.9µg of **2**, 100µg of **1** and 145.2µg of **2,** 100µg of **1** and 290.4µg of **2,** 200µg, of **1** and 18.2µg of **2**, 200µg of **1** and 36.3µg of **2,** 200µg of **1** and 72.6µg of **2**, 200µg of **1** and 108.9µg of **2**, 200µg of **1** and 145.2µg of **2**, 200µg of **1** and 290.4µg of **2**, 300µg of **1** and 18.2µg of **2**, 300µg of **1** and 36.3µg of **2**, 300µg of **1** and 72.6µg of **2**, 300µg of **1** and 108.9µg of **2**, 300µg of **1** and 145.2µg of **2**, 300µg of **1** and 290.4µg of **2**, 400µg of **1** and 18.2µg of **2**, 400µg of **1** and 36.3µg of **2**, 400µg of **1** and 72.6µg of **2**, 400µg of **1** and 108.9µg of **2**, 400µg of **1** and 145.2µg of **2**, 400µg of **1** and 290.4µg of **2**, 500µg of **1** and 18.2µg of **2,** 500µg of **1** and 36.3µg of **2**, 500µg of **1** and 72.6µg of **2**, 500µg of **1** and 108.9µg of **2**, 500µg of **1** and 145.2µg of **2**, 500µg of **1** and 290.4µg of **2**, 600µg of **1** and 18.2µg of **2,** 600µg of **1** and 36.3µg of **2**, 600µg of **1** and 72.6µg of **2,** 600µg of **1** and 108.9µg of **2**, 600µg of **1** and 145.2µg of **2**, 600µg of **1** and 290.4µg of **2**, 700µg of **1** and 18.2µg of **2**, 700µg of **1** and 36.3µg of **2**, 700µg of **1** and 72.6µg of **2**, 700µg of **1** and 108.9µg of **2**, 700µg of **1** and 145.2µg of **2,** 700µg of **1** and 290.4µg of **2,** 800µg of **1** and 18.2µg of **2,** 800µg of **1** and 36.3µg of **2,** 800µg of **1** and 72.6µg of **2,** 800µg of **1** and 108.9µg of **2,** 800µg of **1** and 145.2µg of **2,** 800µg of **1** and 290.4µg of **2,** 900µg of **1** and 18.2µg of **2,** 900µg of **1** and 36.3µg of **2,** 900µg of **1** and 72.6µg of **2,** 900µg of **1** and 108.9µg of **2,** 900µg of **1** and 145.2µg of **2,** 900µg of **1** and 290.4µg of **2,** 1000µg of **1** and 18.2µg of **2,** 1000µg of **1** and 36.3µg of **2,** 1000µg of **1** and 72.6µg of **2,** 1000µg of **1** and 108.9µg of **2,** 1000µg of **1** and 145.2µg of **2,** 1000µg of **1** and 290.4µg of **2.**

The aforementioned examples of possible doses applicable for the combinations according to the invention are to be understood as referring to doses per single application. However, these examples are not be understood as excluding the possibility of administering the combinations according to the invention multiple times. Depending on the medical need patients may receive also multiple inhalative applications. As an example patients may receive the combinations according to the invention for instance two or three times (e.g. two or three puffs with a powder inhaler, an MDI etc) in the morning of each treatment day. As the aforementioned dose examples are only to be understood as dose examples per single application (i.e. per puff) multiple application of the combinations according to the invention leads to multiple doses of the aforementioned examples. The application of the combositions according to the invention can be for instance once a day, or depending on the duration of action of the anticholinergic agent twice a day, or once every 2 or 3 days.

Moreover it is emphazised that the aforementioned dose examples are to be understood as examples of metered doses only. In other terms, the aforementioned dose examples are not to be understood as the effective doses of the combinations according to the invention that do in fact reach the lung. It is clear for the person of ordinary skill in the art that the delivered dose to the lung is generally lower than the metered dose of the administered active ingredients.

The active substance combinations of **1** and **2** according to the invention are preferably administered by inhalation. For this purpose, ingredients **1** and **2** have to be made available in forms suitable for inhalation. Inhalable preparations according to the invention include inhalable powders, propellant-containing metered dose aerosols or propellant-free inhalable solutions. Inhalable powders according to the invention containing the combination of active substances 1 and 2 may consist of the active substances on their own or of a mixture of the active substances with physiologically acceptable excipients. Within the scope of the present invention, the term carrier may optionally be used instead of the term excipient. Within the scope of the present invention, the term propellant-free inhalable solutions also includes concentrates or sterile inhalable solutions ready for use. The preparations according to the invention may contain the combination of active substances **1** and **2** either together in one formulation or in two separate formulations. These formulations which may be used within the scope of the present invention are described in more detail in the next part of the specification.

### A) Inhalable powder containing the combinations of active substances 1 and 2 according to the invention:

The inhalable powders according to the invention may contain **1** and **2** either on their own or in admixture with suitable physiologically acceptable excipients.
If the active substances 1 and 2 are present in admixture with physiologically acceptable excipients, the following physiologically acceptable excipients may be used to prepare these inhalable powders according to the invention: monosaccharides (e.g. glucose or arabinose), disaccharides (e.g. lactose, saccharose, maltose, trehalose), oligo- and polysaccharides (e.g. dextran), polyalcohols (e.g. sorbitol, mannitol, xylitol), cyclodextrines (e.g. α-cyclodextrine, β-cyclodextrine, χ-cyclodextrine, methyl-β-cyclodextrine, hydroxypropyl-β-cyclodextrine), salts (e.g. sodium chloride, calcium carbonate) or mixtures of these excipients with one another. Preferably, mono- or disaccharides are used, while the use of lactose, trehalose or glucose is preferred, particularly, but not exclusively, in the form of their hydrates.

Within the scope of the inhalable powders according to the invention the excipients have a maximum average particle size of up to 250µm, preferably between 10 and 150µm, most preferably between 15 and 80µm. It may sometimes seem appropriate to add finer excipient fractions with an average particle size of 1 to 9µm to the excipient mentioned above. These finer excipients are also selected from the group of possible excipients listed hereinbefore. Finally, in order to prepare the inhalable powders according to the invention, micronised active substance **1** and **2**, preferably with an average particle size of 0.5 to 10µm, more preferably from 1 to 6µm, is added to the excipient mixture. Processes for producing the inhalable powders according to the invention by grinding and micronising and by finally mixing the ingredients together are known from the prior art. The inhalable powders according to the invention may be prepared and administered either in the form of a single powder mixture which contains both **1** and **2** or in the form of separate inhalable powders which contain only **1** or **2.**

The inhalable powders according to the invention may be administered using inhalers known from the prior art. Inhalable powders according to the invention which contain one or more physiologically acceptable excipients in addition to **1** and **2** may be administered, for example, by means of inhalers which deliver a single dose from a supply using a measuring chamber as described in US 4570630A, or by other means as described in
DE 36 25 685 A. The inhalable powders according to the invention which contain **1** and **2** optionally in conjunction with a physiologically acceptable excipient may be administered, for example, using the inhaler known by the name Turbuhaler^{®} or using inhalers as disclosed for example in EP 237507 A. Preferably, the inhalable powders according to the invention which contain physiologically acceptable excipient in addition to **1** and **2** are packed into capsules (to produce so-called inhalettes) which are used in inhalers as described, for example, in WO 94/28958.

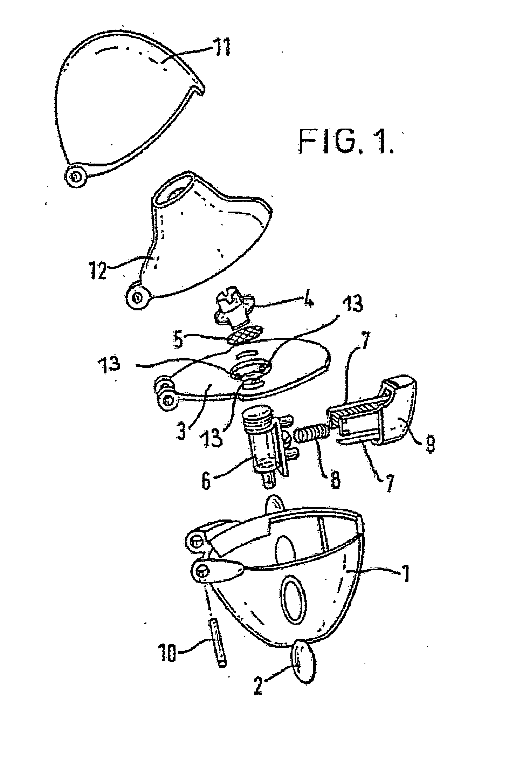

A particularly preferred inhaler for using the pharmaceutical combination according to the invention in inhalettes is shown in Figure 1.
This inhaler (Handyhaler) for inhaling powdered pharmaceutical compositions from capsules is characterised by a housing 1 containing two windows 2, a deck 3 in which there are air inlet ports and which is provided with a screen 5 secured via a screen housing 4, an inhalation chamber 6 connected to the deck 3 on which there is a push button 9 provided with two sharpened pins 7 and movable counter to a spring 8, and a mouthpiece 12 which is connected to the housing 1, the deck 3 and a cover 11 via a spindle 10 to enable it to be flipped open or shut, as well as airholes 13 for adjusting the flow resistance.

If the inhalable powders according to the invention are packed into capsules (inhalers) for the preferred use described above, the quantities packed into each capsule should be 1 to 30mg per capsule. These capsules contain, according to the invention, either together or separately, the doses of **1** and **2** or **2'** mentioned hereinbefore for each single dose.

### B) Propellant gas-driven inhalation aerosols containing the combinations of active substances 1 and 2:

Inhalation aerosols containing propellant gas according to the invention may contain substances **1** and **2** dissolved in the propellant gas or in dispersed form. **1** and **2** may be present in separate formulations or in a single preparation, in which **1** and **2** are either both dissolved, both dispersed or only one component is dissolved and the other is dispersed. The propellant gases which may be used to prepare the inhalation aerosols according to the invention are known from the prior art. Suitable propellant gases are selected from among hydrocarbons such as n-propane, n-butane or isobutane and halohydrocarbons such as fluorinated derivatives of methane, ethane, propane, butane, cyclopropane or cyclobutane. The propellant gases mentioned above may be used on their own or in mixtures thereof. Particularly preferred propellant gases are halogenated alkane derivatives selected from TG11, TG12, TG134a (1,1,1,2-tetrafluoroethane) and TG227 (1,1,1,2,3,3,3-heptafluoropropane) and mixtures thereof, of which the propellant gases TG134a, TG227 and mixtures thereof are preferred.

The propellant-driven inhalation aerosols according to the invention may also contain other ingredients such as co-solvents, stabilisers, surfactants, antioxidants, lubricants and pH adjusters. All these ingredients are known in the art.

The inhalation aerosols containing propellant gas according to the invention may contain up to 5 wt.-% of active substance **1** and/or **2**. Aerosols according to the invention contain, for example, 0.002 to 5 wt.-%, 0.01 to 3 wt.-%, 0.015 to 2 wt.-%, 0.1 to 2 wt.-%, 0.5 to 2 wt.-% or 0.5 to 1 wt.-% of active substance **1** and/or **2.**

If the active substances **1** and/or **2** are present in dispersed form, the particles of active substance preferably have an average particle size of up to 10µm, preferably from 0.1 to 6µm, more preferably from 1 to 5µm.

The propellant-driven inhalation aerosols according to the invention mentioned above may be administered using inhalers known in the art (MDIs = metered dose inhalers).

Accordingly, in another aspect, the present invention relates to pharmaceutical compositions in the form of propellant-driven aerosols as hereinbefore described combined with one or more inhalers suitable for administering these aerosols. In addition, the present invention relates to inhalers which are characterised in that they contain the propellant gas-containing aerosols described above according to the invention. The present invention also relates to cartridges fitted with a suitable valve which can be used in a suitable inhaler and which contain one of the above-mentioned propellant gas-containing inhalation aerosols according to the invention. Suitable cartridges and methods of filling these cartridges with the inhalable aerosols containing propellant gas according to the invention are known from the prior art.

### C) Propellant-free inhalable solutions or suspensions containing the combinations of active substances 1 and 2 according to the invention:

Propellant-free inhalable solutions and suspensions according to the invention contain, for example, aqueous or alcoholic, preferably ethanolic solvents, optionally ethanolic solvents mixed with aqueous solvents. If aqueous/ethanolic solvent mixtures are used the relative proportion of ethanol compared with water is not limited but preferably the maximum is up to 70 percent by volume, more particularly up to 60 percent by volume of ethanol. The remainder of the volume is made up of water. The solutions or suspensions containing **1** and **2**, separately or together, are adjusted to a pH of 2 to 7, preferably 2 to 5, using suitable acids. The pH may be adjusted using acids selected from inorganic or organic acids. Examples of particularly suitable inorganic acids include hydrochloric acid, hydrobromic acid, nitric acid, sulphuric acid and/or phosphoric acid. Examples of particularly suitable organic acids include ascorbic acid, citric acid, malic acid, tartaric acid, maleic acid, succinic acid, fumaric acid, acetic acid, formic acid and/or propionic acid etc. Preferred inorganic acids are hydrochloric and sulphuric acids. It is also possible to use the acids which have already formed an acid addition salt with one of the active substances. Of the organic acids, ascorbic acid, fumaric acid and citric acid are preferred. If desired, mixtures of the above acids may be used, particularly in the case of acids which have other properties in addition to their acidifying qualities, e.g. as flavourings, antioxidants or complexing agents, such as citric acid or ascorbic acid, for example. According to the invention, it is particularly preferred to use hydrochloric acid to adjust the pH.

According to the invention, the addition of editic acid (EDTA) or one of the known salts thereof, sodium editate, as stabiliser or complexing agent is unnecessary in the present formulation. Other embodiments may contain this compound or these compounds. In a preferred embodiment the content based on sodium editate is less than 100mg/100ml, preferably less than 50mg/100 ml, more preferably less than 20mg/100 ml. Generally, inhalable solutions in which the content of sodium editate is from 0 to 10mg/100ml are preferred.

Co-solvents and/or other excipients may be added to the propellant-free inhalable solutions according to the invention. Preferred co-solvents are those which contain hydroxyl groups or other polar groups, e.g. alcohols - particularly isopropyl alcohol, glycols - particularly propyleneglycol, polyethyleneglycol, polypropyleneglycol, glycolether, glycerol, polyoxyethylene alcohols and polyoxyethylene fatty acid esters. The terms excipients and additives in this context denote any pharmacologically acceptable substance which is not an active substance but which can be formulated with the active substance or substances in the pharmacologically suitable solvent in order to improve the qualitative properties of the active substance formulation: Preferably, these substances have no pharmacological effect or, in connection with the desired therapy, no appreciable or at least no undesirable pharmacological effect. The excipients and additives include, for example, surfactants such as soya lecithin, oleic acid, sorbitan esters, such as polysorbates, polyvinylpyrrolidone, other stabilisers, complexing agents, antioxidants and/or preservatives which guarantee or prolong the shelf life of the finished pharmaceutical formulation, flavourings, vitamins and/or other additives known in the art. The additives also include pharmacologically acceptable salts such as sodium chloride as isotonic agents.

The preferred excipients include antioxidants such as ascorbic acid, for example, provided that it has not already been used to adjust the pH, vitamin A, vitamin E, tocopherols and similar vitamins and provitamins occurring in the human body.

Preservatives may be used to protect the formulation from contamination with pathogens. Suitable preservatives are those which are known in the art, particularly cetyl pyridinium chloride, benzalkonium chloride or benzoic acid or benzoates such as sodium benzoate in the concentration known from the prior art. The preservatives mentioned above are preferably present in concentrations of up to 50mg/100ml, more preferably between 5 and 20mg/100ml.

Preferred formulations contain, in addition to the solvent water and the combination of active substances **1** and **2**, only benzalkonium chloride and sodium editate. In another preferred embodiment, no sodium editate is present.

The propellant-free inhalable solutions according to the invention are administered in particular using inhalers of the kind which are capable of nebulising a small amount of a liquid formulation in the therapeutic dose within a few seconds to produce an aerosol suitable for therapeutic inhalation. Within the scope of the present invention, preferred inhalers are those in which a quantity of less than 100µL, preferably less than 50µL, more preferably between 20 and 30µL of active substance solution can be nebulised in preferably one spray action to form an aerosol with an average particle size of less than 20µm, preferably less than 10µm, in such a way that the inhalable part of the aerosol corresponds to the therapeutically effective quantity.

An apparatus of this kind for propellant-free delivery of a metered quantity of a liquid pharmaceutical composition for inhalation is described for example in International Patent Application WO 91/14468 and also in WO 97/12687 (cf. in particular Figures 6a and 6b). The nebulisers (devices) described therein are known by the name Respimat®.
This nebuliser (Respimat®) can advantageously be used to produce the inhalable aerosols according to the invention containing the combination of active substances **1** and **2**. Because of its cylindrical shape and handy size of less than 9 to 15 cm long and 2 to 4 cm wide, this device can be carried at all times by the patient. The nebuliser sprays a defined volume of pharmaceutical formulation using high pressures through small nozzles so as to produce inhalable aerosols.

The preferred atomiser essentially consists of an upper housing part, a pump housing, a nozzle, a locking mechanism, a spring housing, a spring and a storage container, characterised by
- a pump housing which is secured in the upper housing part and which comprises at one end a nozzle body with the nozzle or nozzle arrangement,
- a hollow plunger with valve body,
- a power takeoff flange in which the hollow plunger is secured and which is located in the upper housing part,
- a locking mechanism situated in the upper housing part,
- a spring housing with the spring contained therein, which is rotatably mounted on the upper housing part by means of a rotary bearing,
- a lower housing part which is fitted onto the spring housing in the axial direction.

The hollow plunger with valve body corresponds to a device disclosed in WO 97/12687. It projects partially into the cylinder of the pump housing and is axially movable within the cylinder. Reference is made in particular to Figures 1 to 4, especially Figure 3, and the relevant parts of the description. The hollow plunger with valve body exerts a pressure of 5 to 60 Mpa (about 50 to 600 bar), preferably 10 to 60 Mpa (about 100 to 600 bar) on the fluid, the measured amount of active substance solution, at its high pressure end at the moment when the spring is actuated. Volumes of 10 to 50 microlitres are preferred, while volumes of 10 to 20 microlitres are particularly preferred and a volume of 15 microlitres per spray is most particularly preferred.

The valve body is preferably mounted at the end of the hollow plunger facing the valve body.

The nozzle in the nozzle body is preferably microstructured, i.e. produced by microtechnology. Microstructured nozzle bodies are disclosed for example in WO 94/07607; reference is hereby made to the contents of this specification, particularly Figure 1 therein and the associated description.

The nozzle body consists for example of two sheets of glass and/or silicon firmly joined together, at least one of which has one or more microstructured channels which connect the nozzle inlet end to the nozzle outlet end. At the nozzle outlet end there is at least one round or non-round opening 2 to 10 microns deep and 5 to 15 microns wide, the depth preferably being 4.5 to 6.5 microns while the length is preferably 7 to 9 microns.
In the case of a plurality of nozzle openings, preferably two, the directions of spraying of the nozzles in the nozzle body may extend parallel to one another or may be inclined relative to one another in the direction of the nozzle opening. In a nozzle body with at least two nozzle openings at the outlet end the directions of spraying may be at an angle of 20 to 160° to one another, preferably 60 to 150°, most preferably 80 to 100°. The nozzle openings are preferably arranged at a spacing of 10 to 200 microns, more preferably at a spacing of 10 to 100 microns, most preferably 30 to 70 microns. Spacings of 50 microns are most preferred. The directions of spraying will therefore meet in the vicinity of the nozzle openings.

The liquid pharmaceutical preparation strikes the nozzle body with an entry pressure of up to 600 bar, preferably 200 to 300 bar, and is atomised into an inhalable aerosol through the nozzle openings. The preferred particle or droplet sizes of the aerosol are up to 20 microns, preferably 3 to 10 microns.

The locking mechanism contains a spring, preferably a cylindrical helical compression spring, as a store for the mechanical energy. The spring acts on the power takeoff flange as an actuating member the movement of which is determined by the position of a locking member. The travel of the power takeoff flange is precisely limited by an upper and lower stop. The spring is preferably biased, via a power step-up gear, e.g. a helical thrust gear, by an external torque which is produced when the upper housing part is rotated counter to the spring housing in the lower housing part. In this case, the upper housing part and the power takeoff flange have a single or multiple V-shaped gear.

The locking member with engaging locking surfaces is arranged in a ring around the power takeoff flange. It consists, for example, of a ring of plastic or metal which is inherently radially elastically deformable. The ring is arranged in a plane at right angles to the atomiser axis. After the biasing of the spring, the locking surfaces of the locking member move into the path of the power takeoff flange and prevent the spring from relaxing. The locking member is actuated by means of a button. The actuating button is connected or coupled to the locking member. In order to actuate the locking mechanism, the actuating button is moved parallel to the annular plane, preferably into the atomiser; this causes the deformable ring to deform in the annular plane. Details of the construction of the locking mechanism are given in WO 97/20590.

The lower housing part is pushed axially over the spring housing and covers the mounting, the drive of the spindle and the storage container for the fluid.
When the atomiser is actuated the upper housing part is rotated relative to the lower housing part, the lower housing part taking the spring housing with it. The spring is thereby compressed and biased by means of the helical thrust gear and the locking mechanism engages automatically. The angle of rotation is preferably a whole-number fraction of 360 degrees, e.g. 180 degrees. At the same time as the spring is biased, the power takeoff part in the upper housing part is moved along by a given distance, the hollow plunger is withdrawn inside the cylinder in the pump housing, as a result of which some of the fluid is sucked out of the storage container and into the high pressure chamber in front of the nozzle.

If desired, a number of exchangeable storage containers which contain the fluid to be atomised may be pushed into the atomiser one after another and used in succession. The storage container contains the aqueous aerosol preparation according to the invention.
The atomising process is initiated by pressing gently on the actuating button. As a result, the locking mechanism opens up the path for the power takeoff member. The biased spring pushes the plunger into the cylinder of the pump housing. The fluid leaves the nozzle of the atomiser in atomised form.

Further details of construction are disclosed in PCT Applications WO 97/12683 and WO 97/20590, to which reference is hereby made.
The components of the atomiser (nebuliser) are made of a material which is suitable for its purpose. The housing of the atomiser and, if its operation permits, other parts as well, are preferably made of plastics, e.g. by injection moulding. For medicinal purposes, physiologically safe materials are used.

Figures 6a/b of WO 97/12687, show the nebuliser (Respimat®) which can advantageously be used for inhaling the aqueous aerosol preparations according to the invention.
Figure 6a of WO 97/12687 shows a longitudinal section through the atomiser with the spring biased while Figure 6b of WO 97/12687 shows a longitudinal section through the atomiser with the spring relaxed.

The upper housing part (51) contains the pump housing (52) on the end of which is mounted the holder (53) for the atomiser nozzle. In the holder is the nozzle body (54) and a filter (55). The hollow plunger (57) fixed in the power takeoff flange (56) of the locking mechanism projects partially into the cylinder of the pump housing. At its end the hollow plunger carries the valve body (58). The hollow plunger is sealed off by means of the seal (59). Inside the upper housing part is the stop (60) on which the power takeoff flange abuts when the spring is relaxed. On the power takeoff flange is the stop (61) on which the power takeoff flange abuts when the spring is biased. After the biasing of the spring the locking member (62) moves between the stop (61) and a support (63) in the upper housing part. The actuating button (64) is connected to the locking member. The upper housing part ends in the mouthpiece (65) and is sealed off by means of the protective cover (66) which can be placed thereon.
The spring housing (67) with compression spring (68) is rotatably mounted on the upper housing part by means of the snap-in lugs (69) and rotary bearing. The lower housing part (70) is pushed over the spring housing. Inside the spring housing is the exchangeable storage container (71) for the fluid (72) which is to be atomised. The storage container is sealed off by the stopper (73) through which the hollow plunger projects into the storage container and is immersed at its end in the fluid (supply of active substance solution).
The spindle (74) for the mechanical counter is mounted in the covering of the spring housing. At the end of the spindle facing the upper housing part is the drive pinion (75). The slider (76) sits on the spindle.

The nebuliser described above is suitable for nebulising the aerosol preparations according to the invention to produce an aerosol suitable for inhalation.
If the formulation according to the invention is nebulised using the method described above (Respimat®) the quantity delivered should correspond to a defined quantity with a tolerance of not more than 25%, preferably 20% of this amount in at least 97%, preferably at least 98% of all operations of the inhaler (spray actuations). Preferably, between 5 and 30 mg of formulation, most preferably between 5 and 20 mg of formulation are delivered as a defined mass on each actuation.

However, the formulation according to the invention may also be nebulised by means of inhalers other than those described above, e.g. jet stream inhalers or other stationary nebulisers.

Accordingly, in a further aspect, the invention relates to pharmaceutical formulations in the form of propellant-free inhalable solutions or suspensions as described above combined with a device suitable for administering these formulations, preferably in conjunction with the Respimat®. Preferably, the invention relates to propellant-free inhalable solutions or suspensions characterised by the combination of active substances **1** and **2** according to the invention in conjunction with the device known by the name Respimat®. In addition, the present invention relates to the above-mentioned devices for inhalation, preferably the Respimat®, characterised in that they contain the propellant-free inhalable solutions or suspensions according to the invention as described hereinbefore.

According to the invention, inhalable solutions which contain the active substances **1** and **2** in a single preparation are preferred. The term "single preparation" also includes preparations which contain the two ingredients **1** and **2** in two-chamber cartridges, as disclosed for example in WO 00/23037. Reference is hereby made to this publication in its entirety.

The propellant-free inhalable solutions or suspensions according to the invention may take the form of concentrates or sterile inhalable solutions or suspensions ready for use, as well as the above-mentioned solutions and suspensions designed for use in a Respimat®. Formulations ready for use may be produced from the concentrates, for example, by the addition of isotonic saline solutions. Sterile formulations ready for use may be administered using energy-operated free-standing or portable nebulisers which produce inhalable aerosols by means of ultrasound or compressed air by the Venturi principle or other principles.

Accordingly, in another aspect, the present invention relates to pharmaceutical compositions in the form of propellant-free inhalable solutions or suspensions as described hereinbefore which take the form of concentrates or sterile formulations ready for use, combined with a device suitable for administering these solutions, characterised in that the device is an energy-operated free-standing or portable nebuliser which produces inhalable aerosols by means of ultrasound or compressed air by the Venturi principle or other methods.

The Examples which follow serve to illustrate the present invention in more detail without restricting the scope of the invention to the following embodiments by way of example.

### Examples of Formulations

The following examples of formulations, which may be obtained analogously to methods known in the art, serve to illustrate the present invention more fully without restricting it to the contents of these examples. In the following exmples the enantiomerically pure form of the preferred enantiomer of the bromide of formula **1** (i.e. formula **1-en**) is used as the active ingredient.

### Inhalable powders:

1)

| **Ingredients** | **µg per capsule** |
|---|---|
| **1-en** (bromide) | 150 |
| Formoterol fumarate dihydrate | 50 |
| Lactose | 12300 |
| **Total** | 12500 |

2)

| **Ingredients** | **µg per capsule** |
|---|---|
| **1-en** (bromide) | 150 |
| Salmeterol xinafoate | 50 |
| Lactose | 12300 |
| **Total** | 12500 |

3)

| **Ingredients** | **µg per capsule** |
|---|---|
| **1-en** (bromide) | 150 |
| Salmeterol xinafoate | 150 |
| Lactose | 12200 |
| **Total** | 12500 |

4)

| **Ingredients** | **µg per capsule** |
|---|---|
| **1-en** (bromide) | 200 |
| Formoterol fumarate dihydrate | 50 |
| Lactose | 24750 |
| **Total** | 25000 |

### B) Propellant-containing inhalable aerosols:

1) Suspension aerosol:

| **Ingredients** | **% by weight** |
|---|---|
| **1-en** (bromide) | 0.050 |
| Salmeterol xinafoate | 0.066 |
| Soya lecithin | 0.2 |
| TG 134a : TG 227 = 2:3 | ad 100 |

2) Suspension aerosol:

| **Ingredients** | **% by weight** |
|---|---|
| **1-en** (bromide) | 0.080 |
| Salmeterol xinafoate | 0.033 |
| Absolute ethanol | 0.5 |
| Isopropyl myristate | 0.1 |
| TG 227 | ad 100 |

3) Suspension aerosol:

| **Ingredients** | **% by weight** |
|---|---|
| **1-en** (bromide) | 0.050 |
| Formoterol fumarate dihydrate | 0.035 |
| Soya lecithin | 0.2 |
| TG 134a : TG 227 = 2:3 | ad 100 |

## Claims

1. Pharmaceutical compositions, **characterised in that** they contain one or more salts of formula **1** 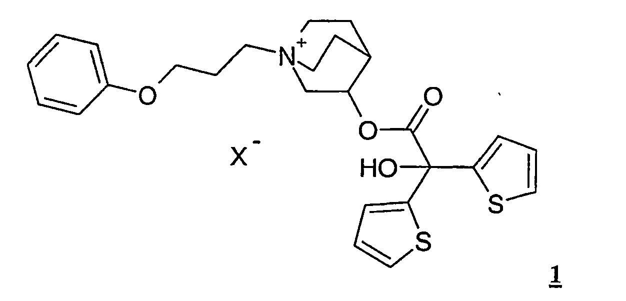 wherein
X⁻ denotes an anion with a single negative charge, preferably an anion selected from the group consisting of fluoride, chloride, bromide, iodide, sulphate, phosphate, methanesulphonate, nitrate, maleate, acetate, citrate, fumarate, tartrate, oxalate, succinate, benzoate and p-toluenesulphonate, optionally in the form of the racemates, the enantiomers, and the hydrates thereof,
combined with one or more betamimetics **(2),** optionally in the form of the enantiomers, mixtures of the enantiomers or in the form of the racemates thereof, optionally in the form of the solvates or hydrates and optionally together with a pharmaceutically acceptable excipient.

2. Pharmaceutical composition according to claim 1, **characterised in that** the active substances **1** and **2** are present either together in a single formulation or in two separate formulations.

3. Pharmaceutical composition according to claim 1 or 2, **characterised in that** the betamimetics **2** are selected from the group consisting of albuterol, bambuterol, bitolterol, broxaterol, carbuterol, clenbuterol, fenoterol, formoterol, hexoprenaline, ibuterol, isoetharine, isoprenaline, levosalbutamol, mabuterol, meluadrine, metaproterenol, orciprenaline, pirbuterol, procaterol, reproterol, rimiterol, ritodrine, salmeterol, salmefamol, soterenot, sulphonterol, tiaramide, terbutaline, tolubuterol, CHF-1035, HOKU-81, KUL-1248, 3-(4-{6-[2-Hydroxy-2-(4-hydroxy-3-hydroxymethyl-phenyl)-ethylamino]-hexyloxy}-butyl)-benzenesulfoneamide, 5-[2-(5,6-Diethyl-indan-2-ylamino)-1-hydroxy-ethyl]-8-hydroxy-1*H*-quinolin-2-one, 4-hydroxy-7-[2-[2-{ [3-(2-phenylethoxy)propyl]sulphonyl} ethyl]-amino} ethyl]-2(3H)-benzothiazolone, 1-(2-fluoro-4-hydroxyphenyl)-2-[4-(1-benzimidazolyl)-2-methyl-2-butylamino]ethanol, 1-[3-(4-methoxybenzyl-amino)-4-hydroxyphenyl]-2-[4-(1-benzimidazolyl)-2-methyl-2-butylamino]ethanol, 1-[2H-5-hydroxy-3-oxo-4H-1,4-benzoxazin-8-yl]-2-[3-(4-N,N-dimethylaminophenyl)-2-methyl-2-propylamino]ethanol,1-[2H-5-hydroxy-3-oxo-4H-1,4-benzoxazin-8-yl]-2-[3-(4-methoxyphenyl)-2-methyl-2-propylamino]ethanol, 1-[2H-5-hydroxy-3-oxo-4H-1,4-benzoxazin-8-yl]-2-[3-(4-n-butyloxyphenyl)-2-methyl-2-propylamino]ethanol, 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol, 5-hydroxy-8-(1-hydroxy-2-isopropylaminobutyl)-2H-1,4-benzoxazin-3-(4H)-one, 1-(4-amino-3-chloro-5-trifluormethylphenyl)-2-tert.-butylamino)ethanol and 1-(4-ethoxycarbonylamino-3-cyano-5-fluorophenyl)-2-(tert.-butylamino)ethanol, optionally in the form of the racemates, the enantiomers, the diastereomers and optionally the pharmacologically acceptable acid addition salts and the hydrates thereof.

4. Pharmaceutical compositions according to one of claims 1 to 3, **characterised in that** the compounds **2** are present in the form of the salts of hydrochloric acid, hydrobromic acid, sulphuric acid, phosphoric acid, methanesulphonic acid, acetic acid, fumaric acid, succinic acid, lactic acid, citric acid, tartaric acid, 1-hydroxy-2-naphthalenecarboxylic acid, 4-phenylcinnamic acid, 5-(2.4-difluorophenyl)salicylic acid or maleic acid or mixtures thereof.

5. Pharmaceutical compositions according to one of claims 1 to 4, **characterised in that** the compounds **2** are selected from the group consisting of bambuterol, bitolterol, carbuterol, clenbuterol, fenoterol, formoterol, hexoprenaline, ibuterol, pirbuterol, procaterol, reproterol, salmeterol, sulphonterol, terbutaline, tolubuterol, 3-(4-{6-[2-Hydroxy-2-(4-hydroxy-3-hydroxymethyl-phenyl)-ethylamino]-hexyloxy }-butyl)-benzenesulfoneamide, 5-[2-(5,6-Diethyl-indan-2-ylamino)-1-hydroxy-ethyl]-8-hydroxy-1*H*-quinolin-2-one, 4-hydroxy-7-[2-{[2-{ [3-(2-phenylethoxy)propyl]sulphonyl }ethyl]-amino}ethyl]-2(3H)-benzothiazolone, 1-(2-fluoro-4-hydroxyphenyl)-2-[4-(1-benzimidazolyl)-2-methyl-2-butylamino]ethanol, 1-[3-(4-methoxybenzyl-amino)-4-hydroxyphenyl]-2-[4-(1-benzimidazolyl)-2-methyl-2-butylamino]ethanol, 1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-hydroxy-3-oxo-4H-1,4-benzoxazin-8-yl]-2-[3-(4-methoxyphenyl)-2-methyl-2-propylamino]ethanol, 1-[2H-5-hydroxy-3-oxo-4H-1,4-benzoxazin-8-yl]-2-[3-(4-n-butyloxyphenyl)-2-methyl-2-propylamino]ethanol, 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol, 5-hydroxy-8-(1-hydroxy-2-isopropylaminobutyl)-2H-1,4-benzoxazin-3-(4H)-one, 1-(4-amino-3-chloro-5-trifluormethylphenyl)-2-tert.-butylamino)ethanol and 1-(4-ethoxycarbonylamino-3-cyano-5-fluorophenyl)-2-(tert.-butylamino)ethanol, optionally in the form of the racemates, the enantiomers, the diastereomers and optionally the pharmacologically acceptable acid addition salts and the hydrates thereof.

6. Pharmaceutical compositions according to one of claims 1 to 5, **characterised in that** the compounds **2** are selected from among fenoterol, formoterol, salmeterol, 3-(4-{6-[2-hydroxy-2-(4-hydroxy-3-hydroxymethyl-phenyl)-ethylamino]-hexyloxy}-butyl)-benzenesulfoneamide, 5-[2-(5,6-Diethyl-indan-2-ylamino)-1-hydroxy-ethyl]-8-hydroxy-1*H*-quinolin-2-one , 1-[3-(4-methoxybenzyl-amino)-4-hydroxyphenyl]-2-[4-(1-benzimidazolyl)-2-methyl-2-butylamino]ethanol, 1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-hydroxy-3-oxo-4H-1,4-benzoxazin-8-yl]-2-[3-(4-methoxyphenyl)-2-methyl-2-propylamino]ethanol, 1-[2H-5-hydroxy-3-oxo-4H-1,4-benzoxazin-8-yl]-2-[3-(4-n-butyloxyphenyl)-2-methyl-2-propylamino]ethanol, 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol, optionally in the form of the racemates, the enantiomers, the diastereomers and optionally the pharmacologically acceptable acid addition salts thereof, and the hydrates thereof.

7. Pharmaceutical composition according to one of claims 1 to 6, **characterised in that** the the compounds of formula **1** are present in form of the enantiomers of formula **1-en**

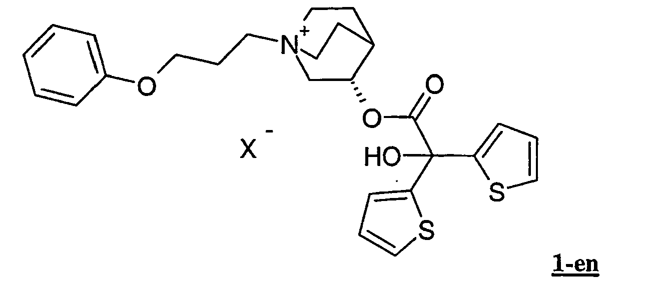

8. Pharmaceutical compositions according to one of claims 1 to 7, **characterised in that** the weight ratios of **1** to **2**' are in a range from about 1:5 to 500:1, preferably 1:10 to 400:1.

9. Pharmaceutical composition according to one of claims 1 to 8, **characterised in that** it is in the form of a preparation suitable for inhalation.

10. Pharmaceutical composition according to claim 9, **characterised in that** it is a preparation selected from among the inhalable powders, propellant-containing metered-dose aerosols and propellant-free inhalable solutions.

11. Pharmaceutical composition according to claim 10, **characterised in that** it is an inhalable powder which contains **1** and **2** in admixture with suitable physiologically acceptable excipients selected from among the monosaccharides, disaccharides, oligo- and polysaccharides, polyalcohols, salts, or mixtures of these excipients with one another.

12. Inhalable powder according to claim 11, **characterised in that** the excipient has a maximum average particle size of up to 250µm, preferably between 10 and 150µm.

13. Pharmaceutical composition according to claim 12, **characterised in that** it is an inhalable powder which contains only the active substances **1** and **2** as its ingredients.

14. Pharmaceutical composition according to claim 10, **characterised in that** it is a propellant-containing inhalable aerosol which contains **1** and **2** in dissolved or dispersed form.

15. Propellant-containing inhalable aerosol according to claim 14, **characterised in that** it contains, as propellant gas, hydrocarbons such as n-propane, n-butane or isobutane or halohydrocarbons such as chlorinated and/or fluorinated derivatives of methane, ethane, propane, butane, cyclopropane or cyclobutane.

16. Propellant-containing inhalable aerosol according to claim 15, **characterised in that** the propellant gas is TG11, TG12, TG134a, TG227 or mixtures thereof, preferably TG134a, TG227 or a mixture thereof.

17. Propellant-containing inhalable aerosol according to one of claims 14 to 16, **characterised in that** it may contain up to 5 % by weight of active substance **1** and/or **2.**

18. Pharmaceutical composition according to claim 10, **characterised in that** it is a propellant-free inhalable solution which contains water, ethanol or a mixture of water and ethanol as solvent.

19. Inhalable solution according to claim 18, **characterised in that** it optionally contains other co-solvents and/or excipients.

20. Inhalable solution according to claim 19, **characterised in that** it contains as co-solvents ingredients which contain hydroxyl groups or other polar groups, e.g. alcohols - particularly isopropyl alcohol, glycols - particularly propyleneglycol, polyethyleneglycol, polypropyleneglycol, glycolether, glycerol, polyoxyethylene alcohols and polyoxyethylene fatty acid esters.

21. Inhalable solutions according to one of claims 19 or 20, **characterised in that** they contain as excipients surfactants, stabilisers, complexing agents, antioxidants and/or preservatives, flavourings, pharmacologically acceptable salts and/or vitamins.

22. Inhalable solutions according to claim 21, **characterised in that** they contain as complexing agents editic acid or a salt of editic acid, preferably sodium edetate.

23. Use of a composition according to one of claims 1 to 22 for preparing a medicament for the treatment of inflammatory or obstructive respiratory complaints, particularly asthma or COPD.

## Patentansprüche

1. Pharmazeutische Zusammensetzungen, **dadurch gekennzeichnet, dass** sie ein Salz oder mehrere Salze der Formel 1 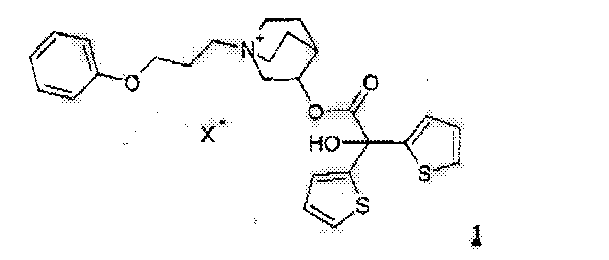 worin
X⁻ ein Anion mit einer einzelnen negativen Ladung, vorzugsweise ein Anion, ausgewählt aus der Gruppe, bestehend aus Fluorid, Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat, bezeichnet, gegebenenfalls in Form der Racemate, Enantiomeren und der Hydrate davon,
kombiniert mit einem oder mehreren Betamimetika (2), gegebenenfalls in Form der Enantiomeren, von Gemischen der Enantiomeren oder in Form der Racemate davon, gegebenenfalls in Form der Solvate oder Hydrate, und gegebenenfalls zusammen mit einem pharmazeutisch annehmbaren Exzipienten bzw. Hilfsstoff, enthalten.

2. Pharmazeutische Zusammensetzung nach Anspruch 1, **dadurch gekennzeichnet, dass** die aktiven Substanzen 1 und 2 entweder zusammen in einer einzelnen Formulierung oder in zwei getrennten Formulierungen vorliegen.

3. Pharmazeutische Zusammensetzung nach Anspruch 1 oder 2, **dadurch gekennzeichnet, dass** die Betamimetika 2 ausgewählt sind aus der Gruppe, bestehend aus Albuterol, Bambuterol, Bitolterol, Broxaterol, Carbuterol, Clenbuterol, Fenoterol, Formoterol, Hexoprenalin, Ibuterol, Isoetharin, Isoprenalin, Levosalbutamol, Mabuterol, Meluadrin, Metaproterenol, Orciprenalin, Pirbuterol, Procaterol, Reproterol, Rimiterol, Ritodrin, Salmeterol, Salmefamol, Soterenot, Sulfonterol, Tiaramid, Terbutalin, Tolubuterol, CHF-1035, HOKU-81, KUL-1248, 3-(4-{6-[2-Hydroxy-2-(4-hydroxy-3-hydroxymethyl-phenyl)-ethylamino]-hexyloxy}-butyl)-benzolsulfonamid, 5-[2-(5,6-Diethyl-indan-2-ylamino)-1-hydroxy-ethyl]-8-hydroxy-1H-chinolin-2-on, 4-Hydroxy-7-[2-{[2-{[3-(2-phenylethoxy)-propyl]-sulfonyl}-ethyl]-amino}-ethyl]-2(3H)-benzothiazolon, 1-(2-Fluor-4-hydroxyphenyl)-2-[4-(1-benzimidazolyl)-2-methyl-2-butylamino]-ethanol, 1-[3-(4-Methoxybenzyl-amino)-4-hydroxyphenyl]-2-[4-(1-benzimidazolyl)-2-methyl-2-butylamino]-ethanol, 1-[2H-5-Hydroxy-3-oxo-4H-benzoxazin-8-yl]-2-[3-(4-N,N-dimethylaminophenyl)-2-methyl-2-propylamino]-ethanol, 1-[2H-5-Hydroxy-3-oxo-4H-1,4-benzoxazin-8-yl]-2-[3-(4-methoxyphenyl)-2-methyl-2-propylamino]-ethanol, 1-[2H-5-Hydroxy-3-oxo-4H-1,4-benzoxazin-8-yl]-2-[3-(4-n-butyloxyphenyl)-2-methyl-2-propylamino]-ethanol, 1-[2H-5-Hydroxy-3-oxo-4H-1,4-benzoxazin-8-yl]-2-{4-[3-(4-methoxyphenyl)-1,2,4-triazol-3-yl]-2-methyl-2-butyl-amino}-ethanol, 5-Hydroxy-8-(1-hydroxy-2-isopropylaminobutyl)-2H-1,4-benzoxazin-3-(4H)-on, 1-(4-Amino-3-chlor-5-trifluormethylphenyl)-2-tert-butylamino)-ethanol und 1-(4-Ethoxycarbonylamino-3-cyano-5-fluorphenyl)-2-(tert-butylamino)-ethanol, gegebenenfalls in Form der Racemate, der Enantiomeren, der Diastereomeren und gegebenenfalls der pharmakologisch annehmbaren Säureadditionssalze und der Hydrate davon.

4. Pharmazeutische Zusammensetzungen nach einem der Ansprüche 1 bis 3, **dadurch gekennzeichnet, dass** die Verbindungen 2 in Form der Salze von Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure, 1-Hydroxy-2-naphthalincarbonsäure, 4-Phenylzimtsäure, 5-(2,4-Difluorphenyl)-salicylsäure oder Maleinsäure oder Gemischen davon vorliegen.

5. Pharmazeutische Zusammensetzungen nach einem der Ansprüche 1 bis 4, **dadurch gekennzeichnet, dass** die Verbindungen 2 ausgewählt sind aus der Gruppe, bestehend aus Bambuterol, Bitolterol, Carbuterol, Clenbuterol, Fenoterol, Formoterol, Hexoprenalin, Ibuterol, Pirbuterol, Procaterol, Reproterol, Salmeterol, Sulfonterol, Terbutalin, Tolubuterol, 3-(4-{6-[2-Hydroxy-2-(4-hydroxy-3-hydroxymethyl-phenyl)-ethylamino]-hexyloxy}-butyl)-benzolsulfonamid, 5-[2-(5,6-Diethyl-indan-2-ylamino)-1-hydroxy-ethyl]-8-hydroxy-1H-chinolin-2-on, 4-Hydroxy-7-[2-{[2-{[3-(2-phenylethoxy)-propyl]-sulfonyl}-ethyl]-amino}-ethyl]-2(3H)-benzothiazolon, 1-(2-Fluor-4-hydroxyphenyl)-2-[4-(1-benzimidazolyl)-2-methyl-2-butylamino]-ethanol, 1-[3-(4-Methoxybenzyl-amino)-4-hydroxyphenyl]-2-[4-(1-benzimidazolyl)-2-methyl-2-butylamino]-ethanol, 1-[2H-5-Hydroxy-3-oxo-4H-1,4-benzoxazin-8-yl]-2-[3-(4-N,N-dimethylaminophenyl)-2-methyl-2-propylamino]-ethanol, 1-[2H-5-Hydroxy-3-oxo-4H-1,4-benzoxazin-8-yl]-2-[3-(4-methoxyphenyl)-2-methyl-2-propylamino]-ethanol, 1-[2H-5-Hydroxy-3-oxo-4H-1,4-benzoxazin-8-yl]-2-[3-(4-n-butyloxyphenyl)-2-methyl-2-propylamino]-ethanol, 1-[2H-5-Hydroxy-3-oxo-4H-1,4-benzoxazin-8-yl]-2-{4-[3-(4-methoxyphenyl)-1,2,4-triazol-3-yl]-2-methyl-2-butylamino}-ethanol, 5-Hydroxy-8-(1-hydroxy-2-isopropylaminobutyl)-2H-1,4-benzoxazin-3-(4H)-on, 1-(4-Amino-3-chlor-5-trifluormethylphenyl)-2-tert-butylamino)-ethanol und 1-(4-Ethoxy-carbonylamino-3-cyano-5-fluorphenyl)-2-(tert-butylamino)-ethanol, gegebenenfalls in Form der Racemate, der Enantiomeren, der Diastereomeren und gegebenenfalls der pharmakologisch annehmbaren Säureadditionssalze und der Hydrate davon.

6. Pharmazeutische Zusammensetzungen nach einem der Ansprüche 1 bis 5, **dadurch gekennzeichnet, dass** die Verbindungen 2 ausgewählt sind aus Fenoterol, Formoterol, Salmeterol, 3-(4-{6-[2-Hydroxy-2-(4-hydroxy-3-hydroxymethyl-phenyl)-ethylamino]-hexyloxy}-butyl)-benzolsulfonamid, 5-[2-(5,6-Diethyl-indan-2-ylamino)-1-hydroxy-ethyl]-8-hydroxy-1H-chinolin-2-on, 1-[3-(4-Methoxybenzyl-amino)-4-hydroxyphenyl]-2-[4-(1-benzimidazolyl)-2-methyl-2-butylamino]-ethanol, 1-[2H-5-Hydroxy-3-oxo-4H-1,4-benzoxazin-8-yl]-2-[3-(4-N,N-dimethylaminophenyl)-2-methyl-2-propylamino]-ethanol, 1-[2H-5-Hydroxy-3-oxo-4H-1,4-benzoxazin-8-yl]-2-[3-(4-methoxyphenyl)-2-methyl-2-propylamino]-ethanol, 1-[2H-5-Hydroxy-3-oxo-4H-1,4-benzoxazin-8-yl]-2-[3-(4-n-butyloxyphenyl)-2-methyl-2-propylamino]-ethanol, 1-[2H-5-Hydroxy-3-oxo-4H-1,4-benzoxazin-8-yl]-2-{4-[3-(4-methoxyphenyl)-1,2,4-triazol-3-yl]-2-methyl-2-butylamino}-ethanol, gegebenenfalls in Form der Racemate, der Enantiomeren, der Diastereomeren und gegebenenfalls der pharmakologisch annehmbaren Säureadditionssalze davon und der Hydrate davon.

7. Pharmazeutische Zusammensetzung nach einem der Ansprüche 1 bis 6, **dadurch gekennzeichnet, dass** die Verbindungen der Formel 1 in Form der Enantiomeren der Formel 1-en vorliegen

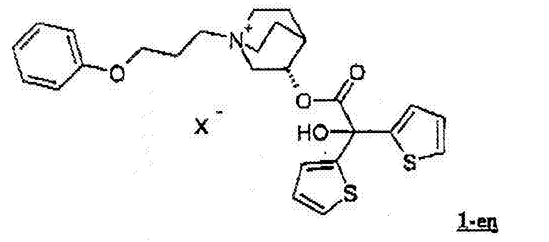

8. Pharmazeutische Zusammensetzungen nach einem der Ansprüche 1 bis 7, **dadurch gekennzeichnet, dass** die Gewichtsverhältnisse von 1 zu 2' im Bereich von etwa 1:5 bis 500:1, vorzugsweise 1:10 bis 400:1 liegen.

9. Pharmazeutische Zusammensetzung nach einem der Ansprüche 1 bis 8, **dadurch gekennzeichnet, dass** sie in Form einer Präparation ist, die zur Inhalation geeignet ist.

10. Pharmazeutische Zusammensetzung nach Anspruch 9, **dadurch gekennzeichnet, dass** sie eine Präparation ist, die ausgewählt ist aus inhalierbaren Pulvern, Treibmittel enthaltenden Dosier-Aerosolen und treibmittelfreien inhalierbaren Lösungen.

11. Pharmazeutische Zusammensetzung nach Anspruch 10, **dadurch gekennzeichnet, dass** sie ein inhalierbares Pulver ist, das 1 und 2 im Gemisch mit geeigneten physiologisch annehmbaren Exzipienten bzw. Hilfsstoffen enthält, die unter den Monosacchariden, Disacchariden, Oligo- und Polysacchariden, Polyalkoholen, Salzen oder Gemischen dieser Exzipienten miteinander ausgewählt sind.

12. Inhalierbares Pulver nach Anspruch 11, **dadurch gekennzeichnet, dass** das Exzipiens bzw. der Hilfsstoff eine maximale durchschnittliche Partikelgröße von bis zu 250 µm, vorzugsweise zwischen 10 und 150 µm, hat.

13. Pharmazeutische Zusammensetzung nach Anspruch 12, **dadurch gekennzeichnet, dass** sie ein inhalierbares Pulver ist, das nur die aktiven Substanzen bzw. Wirkstoffe 1 und 2 als seine Bestandteile enthält.

14. Pharmazeutische Zusammensetzung nach Anspruch 10, **dadurch gekennzeichnet, dass** sie ein Treibmittel enthaltendes inhalierbares Aerosol ist, das 1 und 2 in gelöster oder dispergierter Form enthält.

15. Treibmittel enthaltendes inhalierbares Aerosol nach Anspruch 14, **dadurch gekennzeichnet, dass** es als Treibmittelgas Kohlenwasserstoffe, zum Beispiel n-Propan, n-Butan oder Isobutan oder Halogenkohlenwasserstoffe, zum Beispiel chlorierte und/oder fluorierte Derivate von Methan, Ethan, Propan, Butan, Cyclopropan oder Cyclobutan enthält.

16. Treibmittel enthaltendes inhalierbares Aerosol nach Anspruch 15, **dadurch gekennzeichnet, dass** das Treibmittelgas TG11, TG12, TG134a, TG227 oder Gemische davon, vorzugsweise TG134a, TG227 oder ein Gemisch davon, ist.

17. Treibmittel enthaltendes inhalierbares Aerosol nach einem der Ansprüche 14 bis 16, **dadurch gekennzeichnet, dass** es bis zu 5 Gew.-% an aktiver Substanz bzw. Wirkstoff 1 und/oder 2 enthalten kann.

18. Pharmazeutische Zusammensetzung nach Anspruch 10, **dadurch gekennzeichnet, dass** sie eine treibmittelfreie inhalierbare Lösung ist, die Wasser, Ethanol oder ein Gemisch aus Wasser und Ethanol als Lösungsmittel enthält.

19. Inhalierbare Lösung nach Anspruch 18, **dadurch gekennzeichnet, dass** sie gegebenenfalls andere Co-Lösungsmittel und/oder Exzipienten bzw. Hilfsstoffe enthält.

20. Inhalierbare Lösung nach Anspruch 19, **dadurch gekennzeichnet, dass** sie als Co-Lösungsmittel Bestandteile enthält, welche Hydroxylgruppen oder andere polare Gruppen enthalten, zum Beispiel Alkohole - insbesondere Isopropylalkohol, Glykole - insbesondere Propylenglykol, Polyethylenglykol, Polypropylenglykol, Glykolether, Glycerin, Polyoxyethylenalkohole und Polyoxyethylenfettsäureester.

21. Inhalierbare Lösungen nach einem der Ansprüche 19 oder 20, **dadurch gekennzeichnet, dass** sie als Exzipienten bzw. Hilfsstoffe oberflächenaktive Mittel, Stabilisatoren, Komplexierungsmittel, Antioxidanzien und/oder Konservierungsmittel, Aromen, pharmakologisch annehmbare Salze und/oder Vitamine enthalten.

22. Inhalierbare Lösungen nach Anspruch 21, **dadurch gekennzeichnet, dass** sie als Komplexierungsmittel Edetinsäure oder ein Salz von Edetinsäure, vorzugsweise Natriumedetat, enthalten.

23. Verwendung einer Zusammensetzung nach einem der Ansprüche 1 bis 22 zur Herstellung eines Medikaments für die Behandlung einer Entzündung oder obstruktiver respiratorischer Erkrankungen, insbesondere Asthma oder COPD.

## Revendications

1. Compositions pharmaceutiques, **caractérisées en ce qu'**elles contiennent un ou plusieurs sels de formule **1** 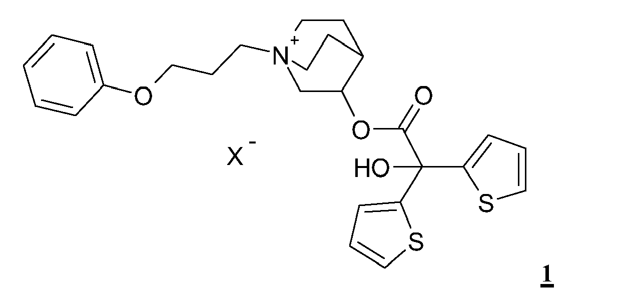 où
X⁻ désigne un anion avec une seule charge négative, de préférence un anion choisi dans le groupe consistant en fluorure, chlorure, bromure, iodure, sulfate, phosphate, méthanesulfonate, nitrate, maléate, acétate, citrate, fumarate, tartrate, oxalate, succinate, benzoate et p-toluènesulfonate, éventuellement sous forme de leurs racémates, de leurs énantiomères et de leurs hydrates.
combinés avec un ou plusieurs bêtamimétiques **(2),** éventuellement sous forme de leurs énantiomères, de mélanges de leurs énantiomères ou sous forme de leurs racémates, éventuellement sous forme des solvates ou hydrates et éventuellement avec un excipient pharmaceutiquement acceptable.

2. Composition pharmaceutique selon la revendication 1, **caractérisée en ce que** les substances actives **1** et **2** sont présentes ensemble dans une seule formulation ou dans deux formulations séparées.

3. Composition pharmaceutique selon la revendication 1 ou 2, **caractérisée en ce que** les bêtamimétiques **2** sont choisis dans le groupe consistant en albuterol, bambuterol, bitolterol, broxaterol, carbuterol, clenbuterol, fenoterol, formoterol, hexoprenaline, ibuterol, isoetharine, isoprenaline, levosalbutamol, mabuterol, meluadrine, metaproterenol, orciprenaline, pirbuterol, procaterol, reproterol, rimiterol, ritodrine, salmeterol, salmefamol, soterenot, sulphonterol, tiaramide, terbutaline, tolubuterol, CHF-1035, HOKU-81, KUL-1248, 3-(4-{6-[2-hydroxy-2-(4-hydroxy-3-hydroxyméthyl-phényl)-éthylamino]-hexyloxy}-butyl)-benzènesulfonamide, 5-[2-(5,6-diéthyl-indan-2-ylamino)-1-hydroxy-éthyl]-8-hydroxy- 1*H*-quinolin-2-one, 4-hydroxy-7-[2- {[2- {[3-(2-phényléthoxy)propyl]-sulfonyl}éthyl]-amino}éthyl]-2(3H)-benzothiazolone, 1-(2-fluoro-4-hydroxy-phényl)-2-[4-(1-benzimidazolyl)-2-méthyl-2-butylamino]éthanol, 1-[3-(4-méthoxy-benzyl-amino)-4-hydroxyphényl]-2-[4-(1-benzimidazolyl)-2-méthyl-2-butylamino]-éthanol, 1-[2H-5-hydroxy-3-oxo-4H-1,4-benzoxazin-8-yl]-2-[3-(4-N,N-diméthyl-aminophényl)-2-méthyl-2-propylamino]éthanol, 1-[2H-5-hydroxy-3-oxo-4H-1,4-benzoxazin-8-yl]-2-[3-(4-méthoxyphényl)-2-méthyl-2-propylamino]éthanol, 1-[2H-5-hydroxy-3-oxo-4H-1,4-benzoxazin-8-yl]-2-[3-(4-n-butyloxyphényl)-2-méthyl-2-propylamino]éthanol, 1-[2H-5-hydroxy-3-oxo-4H-1,4-benzoxazin-8-yl]-2-{4-[3-(4-méthoxyphényl)-1,2,4-triazol-3-yl]-2-méthyl-2-butylamino} éthanol, 5-hydroxy-8-(1-hydroxy-2-isopropylaminobutyl)-2H-1,4-benzoxazin-3-(4H)-one, 1-(4-amino-3-chloro-5-trifluorméthylphényl)-2-tert.-butylamino)éthanol et 1-(4-éthoxy-carbonylamino-3-cyano-5-fluorophényl)-2-(tert.-butylamino)éthanol, éventuellement sous forme de leurs racémates, de leurs énantiomères, de leurs diastéréoisomères et éventuellement de leurs sels d'addition d'acide pharmacologiquement acceptables et de leurs hydrates.

4. Compositions pharmaceutiques selon l'une des revendications 1 à 3, **caractérisées en ce que** les composés **2** sont présents sous forme des sels de l'acide chlorhydrique, de l'acide bromhydrique, de l'acide sulfurique, de l'acide phosphorique, de l'acide méthanesulfonique, de l'acide acétique, de l'acide fumarique, de l'acide succinique, de l'acide lactique, de l'acide citrique, de l'acide tartrique, de l'acide 1-hydroxy-2-naphtalènecarboxylique, de l'acide 4-phénylcinnamique, de l'acide 5-(2.4-difluorophényl)salicylique ou de l'acide maléique ou de leurs mélanges.

5. Compositions pharmaceutiques selon l'une des revendications 1 à 4, **caractérisées en ce que** les composés **2** sont choisis dans le groupe consistant en bambuterol, bitolterol, carbuterol, clenbuterol, fenoterol, formoterol, hexoprenaline, ibuterol, pirbuterol, procaterol, reproterol, salmeterol, sulphonterol, terbutaline, tolubuterol, 3-(4- {6-[2-hydroxy-2-(4-hydroxy-3-hydroxyméthyl-phényl)-éthylamino]-hexyloxy}-butyl)-benzènesulfonamide, 5-[2-(5,6-diéthyl-indan-2-ylamino)-1-hydroxy-éthyl]-8-hydroxy-1*H*-quinolin-2-one, 4-hydroxy-7-[2-{[2-{[3-(2-phényléthoxy)propyl]sulfonyl}éthyl]-amino}éthyl]-2(3H)-benzo-thiazolone, 1-(2-fluoro-4-hydroxyphényl)-2-[4-(1-benzimidazolyl)-2-méthyl-2-butylamino] éthanol, 1-[3-(4-méthoxybenzyl-amino)-4-hydroxyphényl]-2-[4-(1-benzimidazolyl)-2-méthyl-2-butylamino]éthanol, 1-[2H-5-hydroxy-3-oxo-4H-1,4-benzoxazin-8-yl]-2-[3-(4-N,N-diméthylaminophényl)-2-méthyl-2-propylamino]-éthanol, 1-[2H-5-hydroxy-3-oxo-4H-1,4-benzoxazin-8-yl]-2-[3-(4-méthoxy-phényl)-2-méthyl-2-propylamino]éthanol, 1-[2H-5-hydroxy-3-oxo-4H-1,4-benzoxazin-8-yl]-2-[3-(4-n-butyloxyphényl)-2-méthyl-2-propylamino]éthanol, 1-[2H-5-hydroxy-3-oxo-4H-1,4-benzoxazin-8-yl]-2- {4-[3-(4-méthoxyphényl)-1,2,4-triazol-3-yl]-2-méthyl-2-butylamino}éthanol, 5-hydroxy-8-(1-hydroxy-2-isopropyl-aminobutyl)-2H-1,4-benzoxazin-3-(4H)-one, 1-(4-amino-3-chloro-5-trifluoro-méthylphényl)-2-tert.-butylamino)éthanol et 1-(4-éthoxycarbonylamino-3-cyano-5-fluorophényl)-2-(tert.-butylamino)éthanol, éventuellement sous forme de leurs racémates, de leurs énantiomères, de leurs diastéréoisomères et éventuellement de leurs sels d'addition d'acide pharmacologiquement acceptables et de leurs hydrates.

6. Compositions pharmaceutiques selon l'une des revendications 1 à 5, **caractérisées en ce que** les composés **2** sont choisis parmi fenoterol, formoterol, salmeterol, 3-(4-{6-[2-hydroxy-2-(4-hydroxy-3-hydroxyméthyl-phényl)-éthyl-amino]-hexyloxy}-butyl)-benzènesulfoneamide, 5-[2-(5,6-diéthyl-indan-2-yl-amino)-1-hydroxy-éthyl]-8-hydroxy-1*H*-quinolin-2-one, 1-[3-(4-méthoxybenzyl-amino)-4-hydroxyphényl]-2-[4-(1-benzimidazolyl)-2-méthyl-2-butylamino]éthanol, 1-[2H-5-hydroxy-3-oxo-4H-1,4-benzoxazin-8-yl]-2-[3-(4-N,N-diméthylamino-phényl)-2-méthyl-2-propylamino]éthanol, 1-[2H-5-hydroxy-3-oxo-4H-1,4-benzoxazin-8-yl]-2-[3-(4-méthoxyphényl)-2-méthyl-2-propylamino]éthanol, 1-[2H-5-hydroxy-3-oxo-4H-1,4-benzoxazin-8-yl]-2-[3-(4-n-butyloxyphényl)-2-méthyl-2-propylamino]éthanol, 1-[2H-5-hydroxy-3-oxo-4H-1,4-benzoxazin-8-yl]-2-{4-[3-(4-méthoxyphényl)-1 ,2,4-triazol-3-yl]-2-méthyl-2-butylamino éthanol, éventuellement sous forme de leurs racémates, de leurs énantiomères, de leurs diastéréoisomères et éventuellement de leurs sels d'addition d'acide pharmacologiquement acceptables et de leurs hydrates.

7. Composition pharmaceutique selon l'une des revendications 1 à 6, **caractérisée en ce que** les composés de formule **1** sont présents sous forme des énantiomères de formule **1-en**

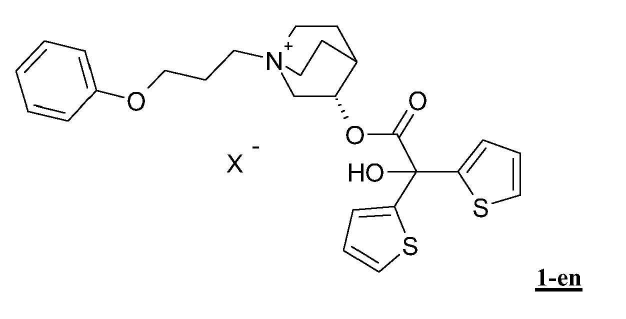

8. Compositions pharmaceutiques selon l'une des revendications 1 à 7, **caractérisées en ce que** les rapports massiques de **1** à **2'** sont dans une plage d'environ 1:5 à 500:1, de préférence de 1:10 à 400:1.

9. Composition pharmaceutique selon l'une des revendications 1 à 8, **caractérisée en ce qu'**elle est sous forme d'une préparation appropriée à l'inhalation.

10. Composition pharmaceutique selon la revendication 9, **caractérisée en ce que** c'est une préparation choisie parmi les poudres inhalables, les aérosols à dose mesurée contenant un propulseur et les solutions inhalables sans propulseur.

11. Composition pharmaceutique selon la revendication 10, **caractérisée en ce que** c'est une poudre inhalable qui contient **1** et **2** en mélange avec des excipients physiologiquement acceptables appropriés choisis parmi les monosaccharides, les disaccharides, les oligo- et polysaccharides, les polyalcools, les sels, ou les mélanges de ces excipients entre eux.

12. Poudre inhalable selon la revendication 11, **caractérisée en ce que** l'excipient a une taille particulaire moyenne maximale pouvant atteindre 250 µm, de préférence entre 10 et 150 µm.

13. Composition pharmaceutique selon la revendication 12, **caractérisée en ce que** c'est une poudre inhalable qui contient seulement les substances actives **1** et **2** comme ingrédients.

14. Composition pharmaceutique selon la revendication 10, **caractérisée en ce que** c'est un aérosol inhalable contenant un propulseur qui contient **1** et **2** sous forme dissoute ou dispersée.

15. Aérosol inhalable contenant un propulseur selon la revendication 14, **caractérisé en ce qu'**il contient, comme gaz propulseur, des hydrocarbures comme le n-propane, le n-butane ou l'isobutane ou des halogénohydrocarbures comme des dérivés chlorés et/ou fluorés du méthane, de l'éthane, du propane, du butane, du cyclopropane ou du cyclobutane.

16. Aérosol inhalable contenant un propulseur selon la revendication 15, **caractérisé en ce que** le gaz propulseur est TG11, TG12, TG134a, TG227 ou des mélanges de ceux-ci, de préférence TG134a, TG227 ou un mélange de ceux-ci.

17. Aérosol inhalable contenant un propulseur selon l'une des revendications 14 à 16, **caractérisé en ce qu'**il peut contenir jusqu'à 5 % en masse de substance active **1** et/ou **2**.

18. Composition pharmaceutique selon la revendication 10, **caractérisée en ce que** c'est une solution inhalable sans propulseur qui contient de l'eau, de l'éthanol ou un mélange d'eau et d'éthanol comme solvant.

19. Solution inhalable selon la revendication 18, **caractérisée en ce qu'**elle contient éventuellement d'autres co-solvants et/ou excipients.

20. Solution inhalable selon la revendication 19, **caractérisée en ce qu'**elle contient comme co-solvants des ingrédients qui contiennent des groupes hydroxyle ou d'autres groupes polaires, par exemple des alcools - en particulier l'alcool isopropylique, des glycols - en particulier le propylèneglycol, le polyéthylèneglycol, le polypropylèneglycol, le glycoléther, le glycérol, des polyoxyéthylène alcools et des esters de polyoxyéthylène et d'acides gras.

21. Solutions inhalables selon l'une des revendications 19 ou 20, **caractérisées en ce qu'**elles contiennent comme excipients des tensioactifs, des stabilisants, des agents complexants, des antioxydants et/ou des conservateurs, des aromatisants, des sels pharmacologiquement acceptables et/ou des vitamines.

22. Solutions inhalables selon la revendication 21, **caractérisées en ce qu'**elles contiennent comme agents complexants de l'acide éditique ou un sel de l'acide éditique, de préférence l'édétate de sodium.

23. Utilisation d'une composition selon l'une des revendications 1 à 22 pour préparer un médicament pour le traitement d'affections inflammatoires ou respiratoires obstructives, en particulier de l'asthme ou de la COPD.
